(19)

Europäisches Patentamt

European Patent Office

Office européen des brevets

(11) **EP 3 981 329 A1**

(12) **EUROPEAN PATENT APPLICATION**

(43) Date of publication:
**13.04.2022 Bulletin 2022/15**

(21) Application number: **21189621.2**

(22) Date of filing: **04.08.2021**

(51) International Patent Classification (IPC):
**A61B 5/145** (2006.01)    **A61B 5/1455** (2006.01)
**A61B 5/00** (2006.01)    **G16H 10/40** (2018.01)

(52) Cooperative Patent Classification (CPC):
**A61B 5/0066; A61B 5/0075; A61B 5/14532;**
**A61B 5/1455; A61B 5/7275**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.10.2020 KR 20200129269**

(71) Applicant: **Samsung Electronics Co., Ltd.**
**Suwon-si, Gyeonggi-do 16677 (KR)**

(72) Inventors:
• **Lee, Jun Ho**
  **Incheon (KR)**
• **Ahn, Sung Mo**
  **Yongin-si (KR)**

(74) Representative: **Grünecker Patent- und Rechtsanwälte PartG mbB Leopoldstraße 4 80802 München (DE)**

(54) **APPARATUS AND METHOD FOR ESTIMATING ANALYTE CONCENTRATION, AND SIGNAL MEASURING APPARATUS**

(57)    An apparatus for non-invasively estimating an analyte concentration is provided. The apparatus for estimating an analyte concentration includes: (1) a signal measurer including: an optical coherence tomography (OCT) device configured to emit an OCT signal to an object and receive the OCT signal reflected or scattered from the object; and a spectrometer configured to emit a spectrometer signal to the object and obtain a spectrum based on the spectrometer signal reflected or scattered from the object, and (2) a processor configured to predict a path length distribution for each wavelength in a predetermined wavelength range, based on the OCT signal, and estimate a concentration of an analyte based on the predicted path length distribution and the obtained spectrum.

FIG. 1

EP 3 981 329 A1

**Description**

BACKGROUND

**1. Field**

Apparatuses and methods consistent with example embodiments relate to non-invasively estimating an analyte concentration.

**2. Description of the Related Art**

Diabetes is a chronic disease that causes various complications and can be hardly cured, such that people with diabetes are advised to check their blood glucose regularly to prevent complications. In particular, when insulin is administered to control blood glucose, the blood glucose levels have to be closely monitored to avoid hypoglycemia and control insulin dosage. An invasive method of finger pricking is generally used to measure blood glucose levels. However, while the invasive method may provide high reliability in measurement, it may cause pain and inconvenience as well as an increased risk of disease infections due to the use of injection. Recently, research has been conducted on methods of non-invasively estimating an analyte concentration using diffuse spectroscopy without blood sampling.

SUMMARY

According to an aspect of an example embodiment, there is provided an apparatus for estimating an analyte concentration. The apparatus may include a signal measurer including: an optical coherence tomography (OCT) device configured to emit an OCT signal to an object and receive the OCT signal reflected or scattered from the object; and a spectrometer configured to emit a spectrometer signal to the object and obtain a spectrum based on the spectrometer signal reflected or scattered from the object, and a processor configured to predict a path length distribution for each wavelength in a predetermined wavelength range, based on the OCT signal, and estimate a concentration of an analyte based on the predicted path length distribution and the obtained spectrum.

The OCT device may include: a first light source configured to emit a first light; a beam splitter configured to split the first light into a first split light and a second split light; a reference mirror configured to reflect the first split light; and a first photodetector configured to detect coherent light generated from the first split that is reflected from the reference mirror, and the second split light that is emitted to and scattered from the object, and configured to convert the coherent light into the OCT signal.

The spectrometer may include: a second light source configured to emit a second light; and a second photodetector configured to obtain the spectrum by detecting the second light that is emitted by the second light source and scattered from the object.

The processor may be further configured to predict the path length distribution for each wavelength based on a scattering coefficient and a g-factor for each wavelength.

The processor may be further configured to obtain the scattering coefficient and the g-factor for each penetration depth of the spectrometer signal into the object, based on the OCT signal.

The processor may be further configured to estimate the scattering coefficient for each penetration depth using a scattering coefficient function.

The processor may be further configured to build a path length distribution database (DB) by using Monte Carlo simulation.

Based on the g-factor and the estimated scattering coefficient for each wavelength, the processor may be further configured to obtain the path length distribution for each wavelength from the built path length distribution DB.

The processor may be further configured to obtain an absorption coefficient spectrum based on the path length distribution for each wavelength and the spectrum, and estimate the concentration of the analyte based on the absorption coefficient spectrum.

The processor may be further configured to determine an absorption coefficient for each wavelength, which allows an estimated spectrum, obtained based on the path length distribution for each wavelength and absorption coefficients at each wavelength, to converge on the spectrum obtained from the object.

The processor may be further configured to remove a noise component, including temperature, from the obtained absorption coefficient spectrum.

The processor may be further configured to extract a noise component vector from the obtained absorption coefficient spectrum, based on at least one of Principal Component Analysis and Singular Value Decomposition.

The processor may be further configured to remove noise from the absorption coefficient spectrum by using a noise removal method including a least square method.

The analyte may include at least one of glucose, urea, lactate, triglyceride, total protein, cholesterol, and ethanol.

According to an aspect of another example embodiment, there is provided a method of estimating an analyte concentration, including: measuring an optical coherence tomography (OCT) signal from an object; obtaining a spectrum from the object; predicting a path length distribution for each wavelength in in a predetermined wavelength range, based on the OCT signal; and estimating a concentration of an analyte based on the predicted path length distribution and the obtained spectrum.

The predicting of the path length distribution may include predicting the path length distribution for each wavelength based on a scattering coefficient and a g-factor for each wavelength.

The predicting of the path length distribution may include obtaining the scattering coefficient and the g-factor for each penetration depth based on the OCT signal.

The predicting of the path length distribution may further include estimating the scattering coefficient for each wavelength based on the scattering coefficient for each penetration depth using a scattering coefficient function.

The predicting of the path length distribution may further include building a path length distribution database (DB) by using Monte Carlo simulation.

The predicting of the path length distribution may further include, based on the scattering coefficient and the g-factor for each wavelength, obtaining the path length distribution for each wavelength from the path length distribution DB.

The estimating of the concentration of the analyte may include obtaining an absorption coefficient spectrum based on the path length distribution for each wavelength and the spectrum, and estimating the concentration of the analyte based on the obtained absorption coefficient spectrum.

The estimating of the concentration of the analyte may include determining an absorption coefficient for each wavelength, which allows an estimated spectrum, obtained based on the path length distribution for each wavelength and absorption coefficients at each wavelength, to converge on the spectrum obtained from the object.

The estimating of the concentration of the analyte may include removing a noise component, including temperature, from the obtained absorption coefficient spectrum.

The removing of the noise component may include extracting a noise component vector based on at least one of Principal Component Analysis and Singular Value Decomposition.

The removing of the noise component may include removing noise from the absorption coefficient spectrum by using a noise removal method including a least square method.

According to an aspect of an example embodiment, there is provided a signal measuring apparatus that includes an optical coherence tomography (OCT) device including: a first light source configured to emit a first light; a beam splitter configured to split the first light into a first split light and a second split light; a reference mirror configured to reflect the first split light; and a first photodetector configured to detect coherent light generated from the first split light that is reflected from the reference mirror, and the second split light that is emitted to and scattered from an object, and configured to convert the detected coherent light into an OCT signal; and a spectrometer including: a second light source configured to emit a second light; and a second photodetector configured to obtain a spectrum by detecting the second light that is emitted by the second light source and scattered from the object.

The OCT device may include: a data acquisition (DAQ) device configured to collect the OCT signal output from the first photodetector; and an analog-to-digital (A/D) converter configured to convert an analog signal that is generated by the DAQ device, into a digital signal.

BRIEF DESCRIPTION OF THE DRAWINGS

The above and/or other aspects will be more apparent by describing certain example embodiments, with reference to the accompanying drawings, in which:

FIG. 1 is a block diagram illustrating an apparatus for estimating an analyte concentration according to an embodiment of the present disclosure;
FIG. 2 is a block diagram illustrating a signal measurer according to an embodiment of the present disclosure;
FIGS. 3A and 3B are diagrams explaining an example of extracting a scattering coefficient for each depth;
FIGS. 4A to 4C are diagrams explaining an example of obtaining a path length distribution for each depth;
FIGS. 5A and 5B are diagrams explaining an example of extracting an absorption coefficient for each wavelength;
FIG. 6 is a diagram illustrating an example of a spectrum of an extracted absorption coefficient;
FIG. 7 is a block diagram illustrating an apparatus for estimating an analyte concentration according to another embodiment of the present disclosure;
FIG. 8 is a flowchart illustrating a method of estimating an analyte concentration according to an embodiment of the present disclosure;
FIG. 9 is a diagram illustrating an example of the predicting of the path length distribution for each wavelength in operation 830;
FIG. 10 is a block diagram illustrating an electronic device including an apparatus for estimating an analyte concentration;
FIG. 11 is a diagram illustrating an example of a wristwatch-type electronic device including an apparatus for estimating an analyte concentration;
FIG. 12 is a diagram illustrating an example of an electronic device implemented as a mobile device and including an apparatus for estimating an analyte concentration; and
FIG. 13 is a diagram illustrating an example of an electronic device implemented as an ear wearable device and including an apparatus for estimating an analyte concentration.

DETAILED DESCRIPTION

Example embodiments for estimating an analyte concentration are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., should be understood as a unit that performs at least one function or operation and that may be embodied as hardware, software, or a combination thereof.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

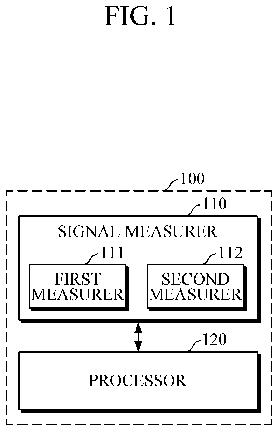

FIG. 1 is a block diagram illustrating an apparatus for estimating an analyte concentration according to an embodiment of the present disclosure.

Referring to FIG. 1, the apparatus 100 for estimating an analyte concentration includes a signal measurer 110 and a processor 120.

The signal measurer 110 may include: a first measurer 111 that may measure an optical coherence tomography (OCT) signal to obtain a scattering coefficient and a g-factor for each depth from an object which is a scattering medium; and a second measurer 112 that may measure a spectrum based on light reflected or scattered from the object. The first measurer 111 and the second measurer 112 are mutually coupled and may be configured to share one lens to measure the same portion of the object. Signals measured by the respective measurers 11 and 112 may be synchronized to be stored in a storage or may be output to the processor 120. The signal measurer 110 may be manufactured as an independent device, which is physically separate from the processor 120, and may transmit the measured signals to an external device, having an algorithm for estimating an analyte concentration, through wired or wireless communications. The first measurer 111 may be also referred to as an OCT scanner or an OCT device, and the second measurer 112 may be also referred to as a spectrometer.

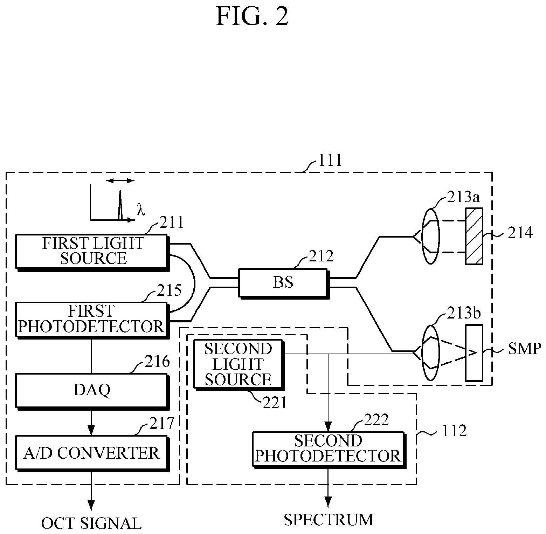

FIG. 2 is a block diagram illustrating a signal measurer according to an embodiment of the present disclosure.

Referring to FIG. 2, the first measurer 111 may include a first light source 211 emitting light onto an object, an interferometer generating coherent light for measuring an OCT signal from the object, a first photodetector 215 detecting the coherent light and converting the detected coherent light into the OCT signal, a data acquisition (DAQ) device 216, and an analog-to-digital (A/D) converter 217. The interferometer may include a beam splitter 212, lenses 213a and 213b, and a reference mirror 214.

The first light source 211 may emit light of a wavelength used for measuring the OCT signal. In particular, the wavelength used for measuring the OCT signal may be in a range of 850 nm or less or in a range of 1300 nm or less, in which a relatively less amount of water is absorbed so that the OCT signal may be measured in an environment where a scattering coefficient is much greater than an absorption coefficient. In the wavelength of the light used for measuring the OCT, the scattering coefficient of the light may be greater than the absorption coefficient of the light by a present value. However, the wavelength range is not limited thereto, and light in two or more wavelength ranges may be emitted.

The beam splitter 212 may split the light emitted by the first light source 211, and may allow a portion of the split light to be incident on the reference mirror 214, and the rest of the split light to be incident on a measured portion of the object SMP. The beam splitter 212 may split the light emanating from the first light source 211 at a predetermined ratio of, for example, 50:50, without optical loss, and may include an optical coupler. Some of the light split by the beam splitter 212 may pass through the first lens 213a to be incident on the reference mirror 214, and the rest of the split light may pass through the second lens 213b to be incident on the object SMP.

Some of the light split by the beam splitter 212 is reflected from the reference mirror 214, and the rest of the split light reacts in various ways, such as being transmitted into or scattered or reflected from the surface or internal organs of the object. First light, reflected from the reference mirror 214, and second light reacting in various ways, such as being transmitted into or scattered or reflected from the object (hereinafter collectively referred to as "scattered") are incident on the beam splitter 212 again, and the beam splitter 212 may generate coherent light from the incident first and second light, and may transmit the coherent light to the first photodetector 215.

The first photodetector 215 may detect the coherent light, generated from the first light and the second light, and may convert the coherent light into an electric signal. The first photodetector 215 may include one or more pixels, each of which includes a photo diode, a photo transistor, a photogate, a pinned photo diode, and the like. The OCT signal may include an intensity profile for each penetration depth of the light, and the intensity profile may indicate the intensity of light received by the pixels.

The DAQ device 216 may collect an OCT signal output from the photodetector 215, and may amplitude the OCT signal. The A/D converter 217 may convert the OCT signal in analog form, into a digital signal and may output the digital signal. The DAQ device 216 may include a memory for storing the OCT signal and/or an amplifier for amplifying the OCT signal.

Referring to FIG. 2, the second measurer 112 may include a second light source 221 that may emit light onto the object and a second photodetector 222 that may detect light scattered from the object and obtaining a spectrum based on the detected light.

The second light source 221 may emit light of a predetermined wavelength onto the object. Light emitted by the second light source 221 may share the second lens 213b of the first measurer 111, and may be incident onto a portion of the object, which is the same as the portion of the object from which the OCT signal is measured by the first measurer 111. The second light source 221 may emit Near Infrared Rays (NIR), Mid Infrared Rays (MIR), laser light, etc., but the light is not limited thereto, and a light wavelength may vary according to the purpose of measurement, the type of analyte, and the like. The second light source 221 may be formed as a light emitting diode (LED), a laser diode (LD), a phosphor, and the like, but is not limited thereto.

The second light source 221 is not necessarily formed as a single light emitting body, or may be formed as an array of a plurality of light emitting bodies. The respective light emitting bodies of the second light source 221 may be driven sequentially or simultaneously driven in a time-division manner. In this case, the plurality of light emitting bodies may emit light of different wavelengths, so as to measure spectra at different penetration depths. For example, spectra may be obtained at a shallow penetration depth by using a light emitting body of a green wavelength which is a short wavelength, and at a deep penetration depth by using a light emitting body of an infrared wavelength which is a relatively long wavelength. However, the wavelength of the light emitting body is not limited to green and infrared wavelengths, and the light emitting body may have various wavelengths, such as a blue wavelength, a red wavelength, etc., according to the analyte, depth of a measured portion, and the like.

The second photodetector 222 may obtain a spectrum by detecting light scattered from the object and returning through the second lens 213b. The second photodetector 222 may include a photo diode, a photo transistor, a Complementary Metal Oxide Semiconductor (CMOS) image sensor, a charge-coupled device (CCD) image sensor, and the like. The second photodetector 222 is not necessarily formed as a single device, but may be formed as an array of a plurality of devices. The second photodetector 222 may include a prism or a diffraction grating for splitting light scattered from the object.

The second light source 221 and the second photodetector 222 may be spaced apart from each other by a predetermined distance. The second light source 221 and the second photodetector 222 may be separated from each other by two or more distances, so as to measure spectra on two or more different light paths. For example, one LED may be provided, and two or more photodiodes (PD) may be disposed at different distances from the LED. Alternatively, a plurality of LEDs may be provided, and one or more PDs may be disposed at different distances from the respective LEDs. By using a combination of an LED and a PD that are spaced apart from each other by a short distance (e.g., a distance shorter than a first predetermined distance), a spectrum may be obtained from a portion at a shallow penetration depth (e.g., capillaries), and by using a combination of an LED and a PD that are spaced apart from each other by a long distance (e.g., a distance longer than a second predetermined distance), a spectrum may be measured from a portion at a deep penetration depth (e.g., arteriole).

Referring back to FIG. 1, the processor 120 may control the signal measurer 110. The processor 120 may receive the OCT signal and the spectrum from the signal measurer 110, and may estimate an analyte concentration by using the received OCT signal and spectrum. For example, the processor 120 may predict a path length distribution for each wavelength based on the OCT signal. Further, the processor 120 may estimate an analyte concentration from the spectrum by considering the predicted path length distribution for each wavelength.

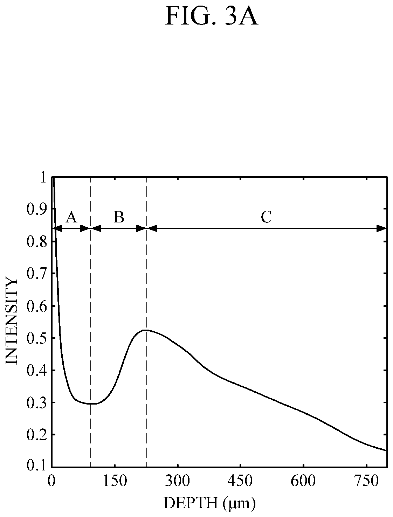

Hereinafter, examples of obtaining a path length distribution for each wavelength and estimating an analyte concentration will be described with reference to FIGS. 3A to 6.

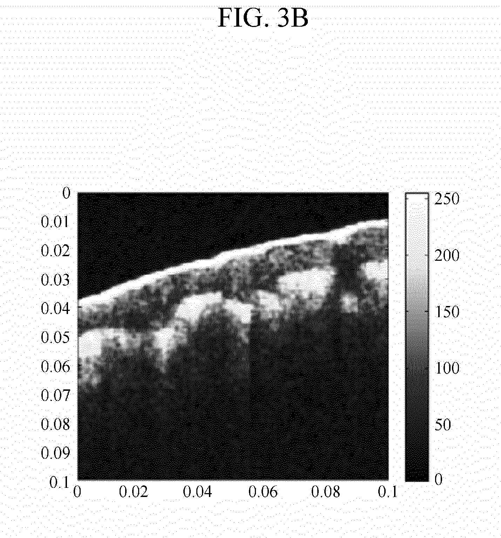

FIGS. 3A and 3B are diagrams explaining an example of extracting a scattering coefficient for each depth.

Referring to FIGS. 3A and 3B, an example of extracting a scattering coefficient and a g-factor for each depth will be described below.

FIG. 3A illustrates an intensity profile (i.e., A-scan data) at each penetration depth of an OCT signal measured by the signal measurer 110. Here, section A represents an epidermis section, section B represents an intermediate section between the epidermis section and a dermis section, and section C represents the dermis section. FIG. 3B illustrates B-scan data of an OCT signal measured by the signal measurer 110.

Based on the intensity profile at each penetration depth of the OCT signal measured by the signal measurer 110, the processor 120 may extract a scattering coefficient and/or a g-factor for each penetration depth. For example, the processor 120 may extract the scattering coefficient and the g-factor for each penetration depth by using the Heterodyne efficiency equation. With respect to a specific depth, the processor 120 may input any one scattering coefficient and g-factor in a specific range into the Heterodyne efficiency equation, and may extract a scattering coefficient and a g-factor, at which an error between an output value of the Heterodyne efficiency equation and an intensity profile measured by the signal measurer 110 is minimized.

The processor 120 may apply the following Heterodyne efficiency Equations 1 and 2 to the light detected by the signal measurer 110:

[Equation 1]

$$\min |I(z) - I_0 \times \Psi(\mu_s, g, z)|$$

Herein, I (z) denotes the intensity of the light for a penetration depth z in the intensity profile at each penetration depth which is measured by the signal measurer 110; $I_0$ denotes an initial incident light intensity; $I_0 \times \Psi(\mu_s, g, z)$ denotes a profile modeled for the penetration depth z by using a scattering coefficient $\mu_s$ and a g-factor g in a specific range, in which $\underline{\Psi(\mu_s, g, z)}$ is a Heterodyne efficiency factor and may be obtained by using the following Equation 2.

[Equation 2]

$$\Psi(z) = \exp(-2\mu_z z) + \frac{4\exp(-\mu_z z)\left[1 - \exp(-\mu_z z)\right]}{1 + w_s^2 / w_H^2} + \left[1 - \exp(-\mu_z z)\right]^2 \frac{w_H^2}{w_s^2}$$

$$w_H^2 = w_0^2\left(A - \frac{B}{f}\right)^2 + \left(\frac{B}{kw_0}\right)^2$$

$$w_s^2 = w_0^2\left(A - \frac{B}{f}\right)^2 + \left(\frac{B}{kw_0}\right)^2 + \left(\frac{2B}{k\rho_0(z)}\right)^2$$

$$\rho_0(z) = \sqrt{\frac{3}{\mu_z z}} \frac{\lambda}{\pi\theta_{rms}}\left(\frac{nB}{z}\right)$$

Herein, A, B, and f denote lens characteristics, in which A may be set to 1, and B may be calculated by adding a value, obtained by dividing the penetration depth z by a refractive index n of a sample, to a distance between the lens and the sample; f denotes a focal length of the lens; z denotes the depth; n denotes the refractive index of the sample; $w_0$ denotes an intensity radius of 1/e of a reference; and $\theta_{rms}$ may be obtained by using a relational expression of g=cos $\theta_{rms}$, in which the scattering coefficient $\mu_s$ and the g-factor g may indicate the input scattering coefficient and g-factor, among g-factors and scattering coefficients in the specific range.

As described above, by changing g-factors and scattering coefficients in the specific range, the processor 120 may model a profile by using the initial incident light $I_0$, and may extract a scattering coefficient and a g-factor, at which the modeled profile $I_0 \times \Psi(\mu_s, g, z)$ converges on the measured profile I(z), as a scattering coefficient and a g-factor for the penetration depth (z). In this manner, the processor 120 may obtain scattering coefficients and g-factors for all of depths.

Upon extracting the scattering coefficient and the g-factor for each penetration depth as described above, the processor 120 may obtain a path length distribution for each wavelength by using the extracted scattering coefficient and g-factor for each penetration depth. For example, the processor 120 may obtain the scattering coefficient for each wavelength by using a Mie scattering equation, such as Equations 1 and 2.

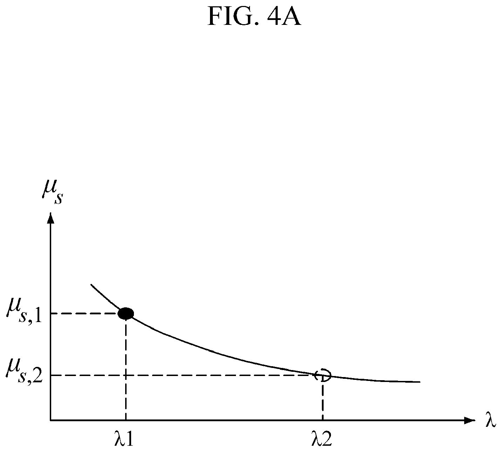

FIGS. 4A to 4C are diagrams explaining an example of obtaining a path length distribution for each depth.

Upon obtaining a scattering coefficient $\mu_{s,1}$ for the penetration depth z by using an OCT signal obtained at a wavelength $\lambda_1$, the processor 120 may derive a relational expression, such as the following Equation 3, by using the Mie scattering equation, and the relational expression may be represented by the graph of FIG. 4A. By using the derived relational expression, the processor 120 may obtain a scattering coefficient $\mu_{s,2}$ to be obtained for a wavelength $\lambda_2$.

[Equation 3]

$$\mu_s(\lambda) = A\,\lambda^{-B}$$

Herein, $\mu_s(\lambda)$ denotes the scattering coefficient to be obtained for the wavelength $\lambda$; B denotes a value preset according to an object and may be set to, for example, -1.5; A denotes a value obtained at the wavelength $\lambda_1$ and the scattering coefficient $\mu_{s,1}$ of the OCT signal. In this case, a relational expression may be derived more accurately compared to a case where the OCT signal is measured at two or more wavelengths.

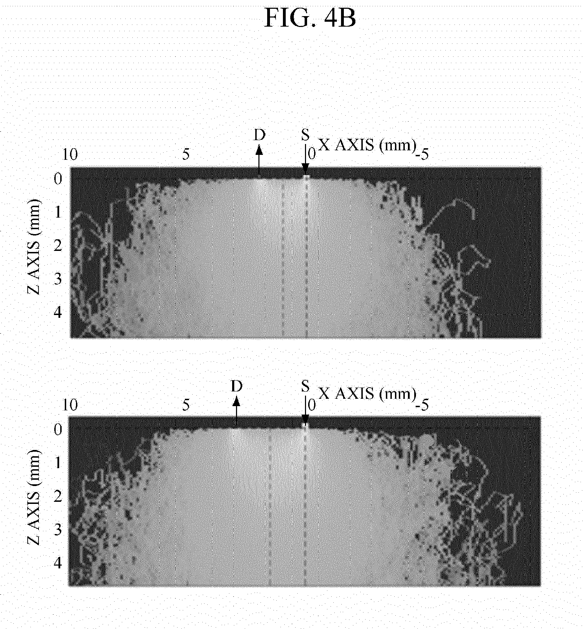

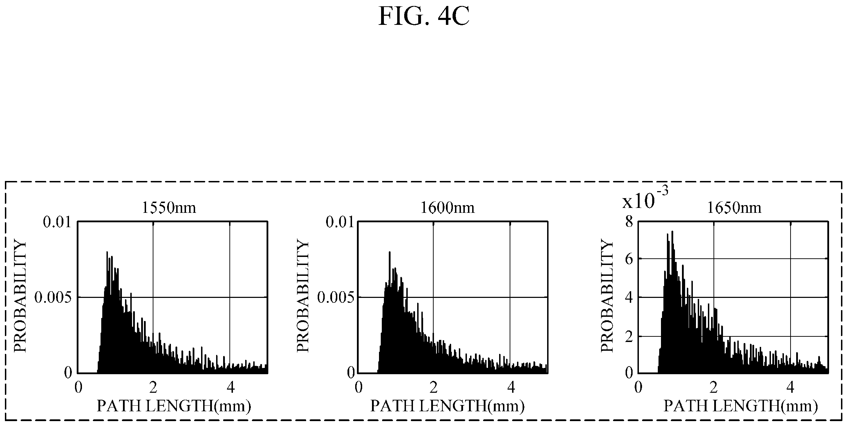

In addition, the processor 120 may build a path length distribution database (DB) for each wavelength by using the g-factors and scattering coefficients in the specific range based on the Monte Carlo Simulation. FIG. 4B illustrates an example of a path length distribution (1) obtained based on the Monte Carlo Simulation for a first distance between a light source and a photodetector, and a path length distribution (2) obtained based on the Monte Carlo Simulation for a second distance, different from the first distance, between a light source and a photodetector. Upon building the path length distribution DB based on the Monte Carlo Simulation, the processor 120 may extract a path length distribution for each wavelength from the path length distribution DB by using the scattering coefficient and the g-factor for each wavelength. FIG. 4C illustrates an example of a path length distribution extracted at the wavelengths of 1550 nm, 1600 nm, and 1650 nm.

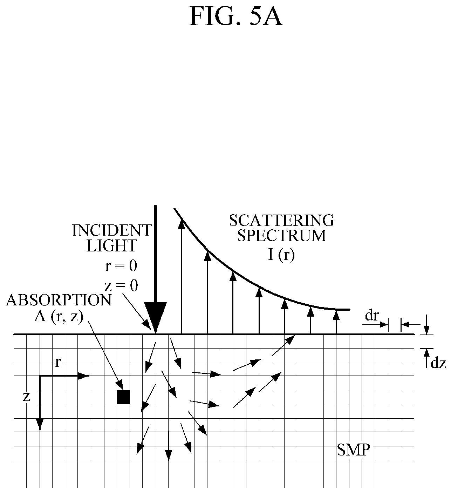

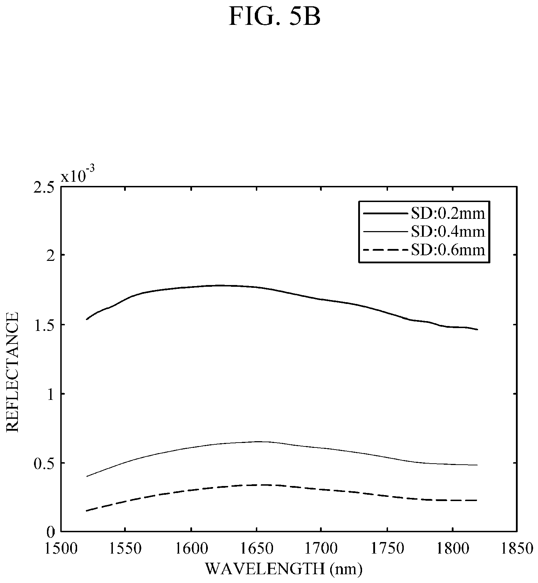

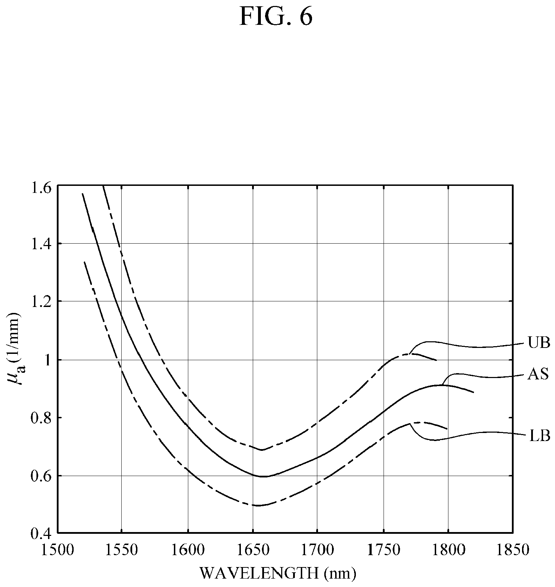

FIGS. 5A and 5B are diagrams explaining an example of extracting an absorption coefficient for each wavelength. FIG. 6 is a diagram illustrating an example of a spectrum of an extracted absorption coefficient.

FIG. 5A illustrates a plurality of path length distributions of light emitted by a light source and passing through tissue to different penetration depths according to tissue characteristics of the object SMP. FIG. 5B illustrates an example of a scattering spectrum according to distances between a light source and a photodetector. As illustrated in FIGS. 5A and 5B, absorbance A of body tissue varies according to a distance r between a light source and a photodetector, a penetration depth z, an incident light intensity, and the like, which indicates that the scattering spectrum I (r) may vary according to various light path lengths.

By using the measured spectrum, the processor 120 may obtain an absorption coefficient spectrum for estimating an analyte concentration, in which as can be seen from FIGS. 5A and 5B, a spectrum intensity may vary according to various light path length distributions, such that the processor 120 may obtain an absorption coefficient for each wavelength by considering path length distributions for each wavelength.

For example, the following Equation 4 represents an example of an equation for calculating an absorption coefficient for each wavelength, and the processor 120 may obtain the absorption coefficient for each wavelength, which satisfies Equation 4. That is, the processor 120 may obtain an absorption coefficient for each wavelength, which allows a spectrum, obtained by using a specific range of absorption coefficients at each wavelength, to converge on the spectrum measured from the object. FIG. 6 illustrates an absorption coefficient spectrum AS obtained by using absorption coefficients within specific ranges LB and UB at each wavelength.

[Equation 4]

$$\min\!\left(I(\lambda) - I_0 \int_0^{\infty} P_l(\lambda)\,\exp(-\mu_a(\lambda)l)\,dl\right)$$

Herein, I ($\lambda$) denotes the intensity of the measured spectrum at the wavelength of $\lambda$; $I_0$ denotes the intensity of

the incident light; $P_l(\lambda)$ denotes a path length distribution at the wavelength of $\lambda$; $l$ denotes a path length; and $\mu_a(\lambda)$ denotes the absorption coefficient to be obtained for the wavelength of $\lambda$.

The processor 120 may remove a noise component, including temperature, from the absorption coefficient spectrum. For example, the processor 120 may extract a noise component vector by using Principal Component Analysis and/or Singular Value Decomposition, and based on the noise component vector, the processor 120 may remove noise from the absorption coefficient spectrum by using a noise removal method such as a least square method.

Upon obtaining the absorption coefficient spectrum as described above, the processor 120 may estimate an analyte concentration by using the absorption coefficient spectrum. In this case, examples of the analyte may include, but is not limited to, glucose, urea, uric acid, lactate, triglyceride, protein, cholesterol, ethanol, and the like. Based on the absorption coefficient spectrum, the processor 120 may estimate an analyte concentration by using linear regression analysis, Partial Least Square (PLS), Classical Least Square (CLS), and the like. In this case, a concentration estimation model, which defines a correlation between the absorption coefficient at each wavelength and the analyte concentration may be predefined by using linear regression analysis and the like.

According to the embodiments described above, an analyte concentration is estimated by obtaining an absorption coefficient for each wavelength in consideration of a path length distribution in a scattering medium, such that accuracy of the estimation may be improved compared to a general method of estimating the analyte concentration based on analysis using the Beer-Lambert Law by assuming that the path length distribution is the same at all wavelengths.

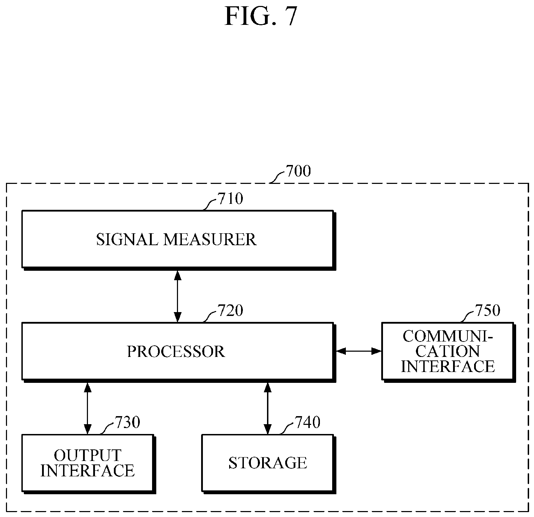

FIG. 7 is a block diagram illustrating an apparatus for estimating an analyte concentration according to another embodiment of the present disclosure.

Referring to FIG. 7, the apparatus 700 for estimating an analyte concentration includes a signal measurer 710, a processor 720, an output interface 730, a storage 740, and a communication interface 750. The signal measurer 710 and the processor 720 are the same as the signal measurer 110 and the processor 120 of FIG. 1, such that a detailed description thereof will be omitted.

The output interface 730 may provide processing results of the processor 720 for a user. For example, the output interface 730 may include a display, and may display an analyte (e.g., an estimated blood glucose value) on the display. In this case, if the estimated blood glucose value falls outside a normal range, the output interface 730 may provide a user with warning information by changing color, line thickness, etc., or displaying the abnormal value along with a normal range, so that the user may easily recognize the abnormal value. Further, the output interface 730 may include a non-visual output module, such as a speaker, a haptic module, etc., and along with or without the visual display, the output interface 610 may provide the user with an estimation result in a non-visual manner by voice, vibrations, tactile sensation, and the like using the haptic module.

The storage 740 may store reference information required for estimating an analyte concentration, and processing results of the signal measurer 710 and/or the processor 720. In this case, the reference information may include user characteristic information, such as a user's age, gender, health condition, and the like. Further, the reference information may include a concentration estimation model. In addition, the reference information may include information, such as algorithms for obtaining a scattering coefficient, a g-factor, the Monte Carlo Simulation, a path length distribution for each wavelength, and the like.

The storage 740 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 750 may communicate with an external device to transmit and receive various data relating to estimating an analyte concentration. The external device may include an information processing device such as a smartphone, a tablet PC, a desktop computer, a laptop computer, and the like. For example, the communication interface 750 may transmit a blood glucose estimation result to a user's smartphone and the like, so that the user may manage and monitor the user's blood glucose by using a device having a relatively high performance. However, the external device is not limited thereto. The communication interface 750 may communicate with the external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G communication, 4G communication, 5G communication, and the like. However, this is merely exemplary and is not intended to be limiting.

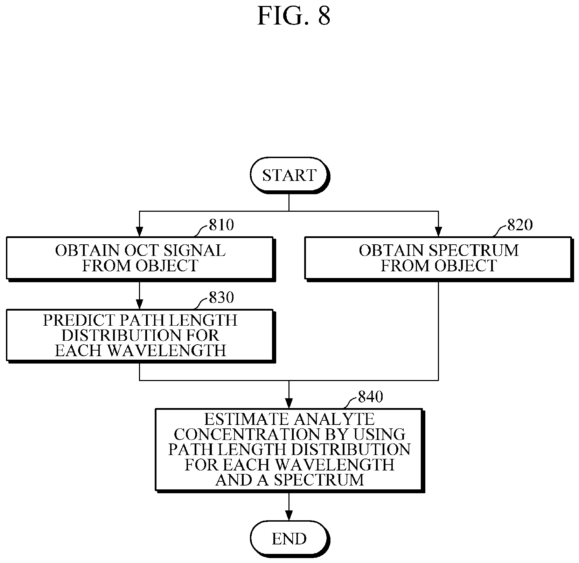

FIG. 8 is a flowchart illustrating a method of estimating an analyte concentration according to an embodiment of the present disclosure.

The method of FIG. 8 is an example of a method of estimating an analyte concentration which is performed by the apparatuses 100 and 700 for estimating an analyte concentration of FIGS. 1 and 7, which is described above in detail, and thus will be briefly described below in order to avoid redundancy.

First, the apparatus for estimating an analyte concentration may obtain an OCT signal from an object by using a first measurer in operation 810, and may obtain a spectrum from the object in operation 820 by emitting light onto the same portion of the object, from which the OCT signal is obtained, and by detecting light scattered from the object, by using a second measurer.

Then, the apparatus for estimating an analyte concentration may predict a path length distribution for each wavelength by using the OCT signal in operation 830.

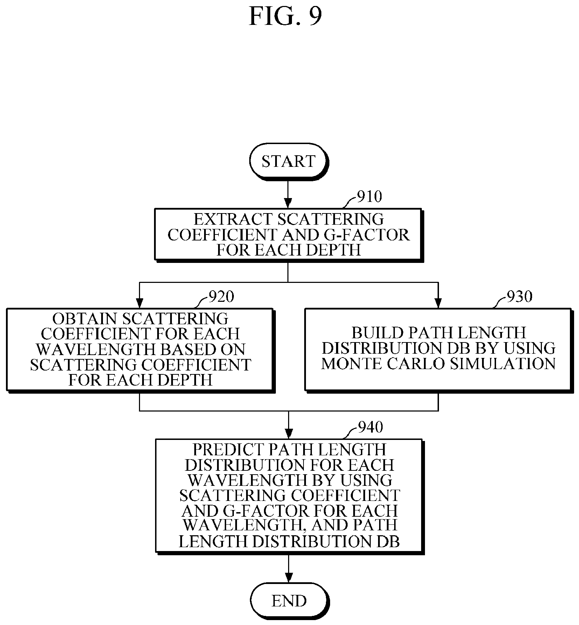

FIG. 9 is a diagram illustrating an example of the operation of predicting the path length distribution for each wavelength in operation 830.

Referring to FIG. 9, an example of the operation of predicting the path length distribution for each wavelength in operation 830 will be described below.

The apparatus for estimating an analyte concentration may extract a scattering coefficient and a g-factor for each penetration depth by using the OCT signal in operation 910. The scattering coefficient and the g-factor for each penetration depth may be obtained by using the Heterodyne efficiency equation, as represented by the above Equations 1 and 2. For example, the apparatus for estimating an analyte concentration may model a profile by changing g-factors and scattering coefficients in a specific range, and may extract a scattering coefficient and a g-factor, at which the modeled profile converges on a measured intensity profile.

Then, the apparatus for estimating an analyte concentration may obtain a scattering coefficient for each wavelength in operation 920 based on the scattering coefficient for each penetration depth obtained in operation 910. For example, the apparatus for estimating an analyte concentration may obtain the scattering coefficient for each wavelength by using Equation 3 derived from the Mie scattering equation.

Subsequently, the apparatus for estimating an analyte concentration may build a path length distribution DB by using scattering coefficients and g-factors in a specific range based on the Monte Carlo Simulation in operation 930. The operation of obtaining the scattering coefficient for each wavelength in operation 920 and the operation of building the path length distribution DB in operation 930 are not necessarily performed in time-sequential order, but any one of the operations may be performed first or both of the operations may be performed at the same time.

Next, the apparatus for estimating an analyte concentration may extract a path length distribution for each wavelength from the path length distribution DB by using the scattering coefficient and the g-factor for each wavelength in operation 940.

Referring back to FIG. 8, upon predicting the path length distribution for each wavelength in operation 830, the apparatus for estimating an analyte concentration may estimate an analyte concentration by using the path length distribution for each wavelength and a spectrum obtained from the object in operation 840. The apparatus for estimating an analyte concentration may obtain an absorption coefficient for each wavelength from the spectrum by using the path length distribution for each wavelength. In this case, the apparatus for estimating an analyte concentration may obtain, for each wavelength, an absorption coefficient which allows a spectrum, obtained by using the path length distribution for each wavelength and an absorption coefficient to be obtained as represented by the above Equation 4, and the spectrum measured in operation 820 to be minimized. Upon obtaining the absorption coefficient spectrum, the apparatus for estimating an analyte concentration may remove noise from the absorption coefficient spectrum by extracting a noise vector such as temperature, and may estimate an analyte concentration based on linear regression analysis and the like by using the absorption coefficient spectrum, from which noise is removed.

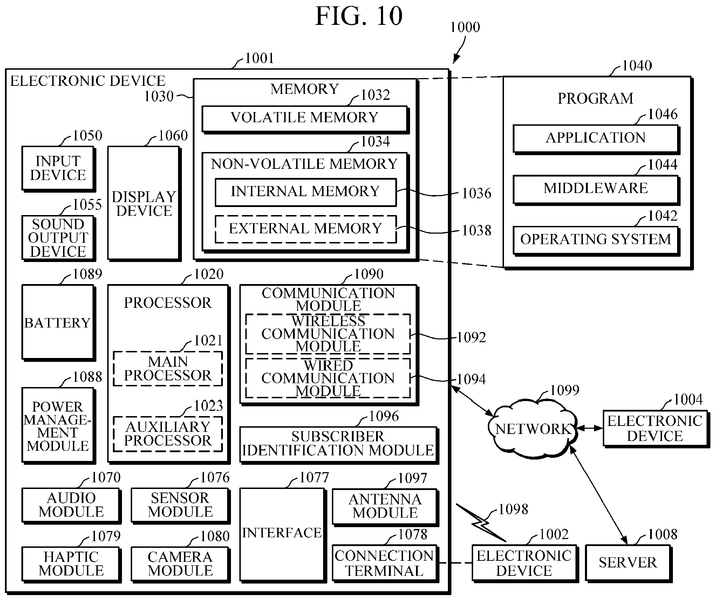

FIG. 10 is a block diagram illustrating an electronic device including an apparatus for estimating an analyte concentration.

Referring to FIG. 10, the electronic device 1001 includes a processor 1020, a memory 1030, an input device 1050, a sound output device 1055, a display device 1060, an audio module 1070, a sensor module 1076, an interface 1077, a haptic module 1079, a camera module 1080, a power management module 1088, a battery 1089, a communication module 1090, a subscriber identification module 1096, and/or an antenna module 1097. At least some of the components may be omitted from the electronic device 1001, and one or more other components may be added in the electronic device 1001.

The aforementioned apparatuses 100 and 700 for estimating an analyte concentration may be implemented as single integrated circuitry to be mounted in the sensor module 1076 of the electronic device 1001, or may be distributed in different components. For example, the signal measurers 110 and 710 of the apparatuses 100 and 700 for estimating an analyte concentration may be included in the sensor module 1076, and the processors 120 and 720 may be included in the processor 1020. Further, the output interface 730 may be distributed in the sound output device 1055, the display device 1060, the audio module 1070, etc.; the storage 740 may be implemented as the memory 1030; and the communication interface 750 may be included in the communication module 1090.

The processor 1020 may execute a program 1040 and the like to control the components of the electronic device 1001 connected to the processor 1020, and may perform various data processing or computation. For example, as part of the data processing or computation, the processor 1020 may load a command and/or data received from the sensor module 1076 or the communication module 1090, etc., in a volatile memory 1032, may process the command and/or the data stored in the volatile memory 1032, and may store resulting data in a non-volatile memory 1034.

The processor 1020 may include a main processor 1021 (e.g., a central processing unit (CPU) or an application processor (AP), etc.), and an auxiliary processor 1023 (e.g., a graphics processing unit (GPU), an image signal processor (ISP), a sensor hub processor, or a communication processor (CP), etc.) that is operable independently from, or in conjunction with, the main processor 1021. The auxiliary processor 1023 may be adapted to consume less power than the main processor 1021, or to be specific to a specified function. The auxiliary processor 1023 may control at least some of functions and/or states related to at least one component, e.g., the display device 1060, the sensor module 1076, the communication module 1090, etc., among the components of the electronic device 1001, instead of the main processor 1021 while the main processor 1021 is in an inactive state (e.g., sleep state), or together with the main processor 1021 while the main processor is in an active state (e.g., application execution state). The auxiliary processor 1023, e.g., an image signal processor, a communication processor, etc., may be implemented as part of another component, e.g., the camera module 1080, the communication module 1090, etc., functionally related to the auxiliary processor 1023.

In response to a user's request for estimating an analyte concentration, the processor 1020 may transmit a control signal to the apparatuses 100 and 700 for estimating an analyte concentration.

The memory 1030 may store various data, for example, software and input data and/or output data for a command related thereto, which are required for the components of the electronic device 1001. The memory 1030 may include a volatile memory 1032 and/or a non-volatile memory 1034.

The program 1040 may be stored as software in the memory 1030, and may include, for example, an operation system (OS) 1042, middleware 1044, and/or an application 1046.

The input device 1050 may receive a command and/or data to be used by another component of the electronic device 1001, from a user, etc., of the electronic device 1001. The input device 1050 may include, for example, a microphone, a mouse, a keyboard, and/or a digital pen (e.g., a stylus pen, etc.).

The sound output device 1055 may output sound signals to the outside of the electronic device 1001. The sound output device 1055 may include, for example, a speaker and/or a receiver. The speaker may be used for general purposes, such as playing multimedia or playing record, and the receiver may be used for incoming calls. The receiver may be implemented separately from, or as part of, the speaker.

The display device 1060 may visually provide information to the outside of the electronic device 1001. The display device 1060 may include, for example, a display, a hologram device, or a projector and control circuitry to control a corresponding one of the display, hologram device, and projector. The display device 1060 may include touch circuity adapted to detect a touch, and/or sensor circuitry (e.g., pressure sensor, etc.) adapted to measure the intensity of force incurred by the touch.

The audio module 1070 may convert a sound into an electrical signal or vice versa. The audio module 1070 may obtain the sound via the input device 1050, or may output the sound via the sound output device 1055, and/or a speaker and/or a headphone of other electronic devices 1002 and 1004 directly or wirelessly connected to and/or the electronic device 1001.

The sensor module 1076 may detect an operating state (e.g., power, temperature, etc.) of the electronic device 1001 or an external environment state (e.g., a state of a user, etc.), and may generate an electrical signal and/or a data value corresponding to the detected state. The sensor module 1076 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor. The apparatuses 100 and 700 for estimating an analyte concentration may be one of biometric sensors included in the sensor module 1076.

The interface 1077 may support one or more specified protocols used by the electronic device 1001 to be directly or wirelessly connected to other electronic devices 1002 and 1004. The interface 1077 may include, for example, a high definition multimedia interface (HDMI), a universal serial bus (USB) interface, a secure digital (SD) card interface, and/or an audio interface.

A connecting terminal 1078 may include a connector via which the electronic device 1001 may be physically connected to other external electronic devices 1002 and 1004. The connecting terminal 1078 may include, for example, an HDMI connector, a USB connector, an SD card connector, and/or an audio connector (e.g., headphone connector, etc.).

A haptic module 1079 may convert an electrical signal into a mechanical stimulus (e.g., vibration, motion, etc.) or electrical stimulus which may be recognized by a user by tactile sensation or kinesthetic sensation. The haptic module 1079 may include, for example, a motor, a piezoelectric element, and/or an electric stimulator.

The camera module 1080 may capture still images or moving images. The camera module 1080 may include a lens assembly having one or more lenses, image sensors, image signal processors, and/or flashes. The lens assembly included in the camera module 1080 may collect light emanating from a subject to be imaged.

The power management module 1088 may manage power supplied to the electronic device 1001. The power management module 1088 may be implemented as part of, for example, a power management integrated circuit (PMIC).

The battery 1089 may supply power to the components of the electronic device 1001. The battery 1089 may include, for example, a primary cell which is not rechargeable, a secondary cell which is rechargeable, or a fuel cell.

The communication module 1090 may support establishment of a direct (e.g., wired) communication channel and/or a wireless communication channel between the electronic device 1001 and other electronic devices 1002 and 1004 within a network environment 1000, and performing of communication via the established communication channel. The communication module 1090 may include one or more communication processors that are operable independently from the processor 1020 and supports a direct communication and/or a wireless communication. The communication module 1090 may include a wireless communication module 1092 (e.g., a cellular communication module, a short-range wireless communication module, or a global navigation satellite system (GNSS) communication module) and/or a wired communication module 1094 (e.g., a local area network (LAN) communication module, a power line communication (PLC) module, etc.). Among these communication modules, a corresponding communication module may communicate with other electronic devices via a first network 1098 (e.g., a short-range communication network, such as Bluetooth, Wi-Fi direct, or infrared data association (IrDA)) or a second network 1099 (e.g., a long-range communication network, such as a cellular network, the Internet, or a computer network (e.g., LAN, wide area network (WAN), etc.). These various types of communication modules 1090 may be implemented as a single chip, etc., or may be implemented as multi chips separate from each other. The wireless communication module 1092 may identify and authenticate the electronic device 1001 in a communication network, such as the first network 1098 or the second network 1099, using subscriber information (e.g., international mobile subscriber identity (IMSI), etc.) stored in the subscriber identification module 1096.

The antenna module 1097 may transmit or receive a signal and/or power to or from an external device. The antenna module 1097 may include an antenna including a radiating element formed of a conductive pattern on a substrate (e.g., polychlorinated biphenyl (PCB), etc.). The antenna module 1097 may include one or a plurality of antennas. In the case where the antenna module 1097 includes a plurality of antennas, at least one antenna appropriate for a communication scheme used in the communication network, such as the first network 1098 and/or the second network 1099, may be selected from among the plurality of antennas by the communication module 1090. Signals or power may be transmitted or received between the communication module 1090 and other electronic device via the selected antenna. In addition to the antenna, other component (e.g., a radio frequency integrated circuit (RFIC), etc.) may be further included as part of the antenna module 1097.

At least some of the above-described components may be mutually connected and may communicate commands, data, etc. therebetween via an inter-peripheral communication scheme (e.g., bus, general purpose input and output (GPIO), serial peripheral interface (SPI), mobile industry processor interface (MIPI), etc.). Commands or data may be transmitted or received between the electronic device 1001 and the external electronic device 1004 via the server 1008 connected to the second network 1099. Other electronic devices 1002 and 1004 may be a device of a same type as, or a different type from, the electronic device 1001. All or some of operations to be executed by the electronic device 1001 may be executed at one or more of other electronic devices 1002, 1004, and 1008. For example, if the electronic device 1001 is required to perform a function or a service automatically, the electronic device 1001, instead of executing the function or the service, may request the one or more other electronic devices to perform at least part of the function or the service. The one or more other electronic devices, which receives the request, may perform at least part of the function or the service requested, or an additional function or an additional service related to the request, and may transmit a result of the performed function or service to the electronic device 1001. To this end, a cloud computing, distributed computing, and/or client-server computing technology may be used.

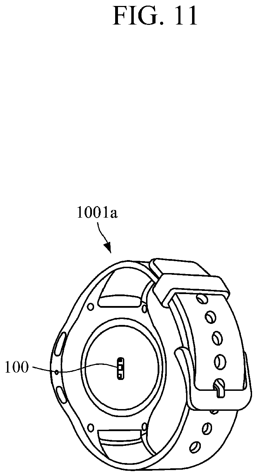

FIGS. 11 to 13 are diagrams illustrating examples of an electronic device in which an apparatus for estimating an analyte concentration is mounted.

Referring to FIG. 11, the electronic device 1001 of FIG. 10 may be implemented as a wristwatch-type wearable device 1001a, and may include a main body and a wrist strap. A display is provided on a front surface of the main body, and may display various application screens, including time information, received message information, and the like. The apparatuses 100 and 700 for estimating an analyte concentration may be disposed on a rear surface of the main body to emit light to a wrist of a user while the wristwatch-type wearable device 1001a is worn around the wrist of the user, and to estimate the concentration of an analyte, such as blood glucose and the like, based on the light reflected or scattered from the wrist.

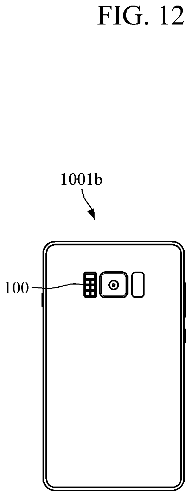

Referring to FIG. 12, the electronic device 1001 of FIG. 10 may be implemented as a mobile device 1001b such as a smartphone.

The mobile device 1001b may include a housing and a display panel. The housing may form an exterior of the mobile device 1001b. The housing has a first surface, on which a display panel and a cover glass may be disposed sequentially, and the display panel may be exposed to the outside through the cover glass. The camera module and/or the infrared sensor of the apparatuses 100 and 700 for estimating an analyte concentration may be disposed on a second surface of the housing. When a user transmits a request for analyte concentration information by executing an application and the like installed in the mobile device 1001b, the mobile device 1001b may estimate the concentration of an analyte by using the apparatus 100 for estimating an analyte concentration, and may provide the estimated concentration information as images and/or sounds to a user.

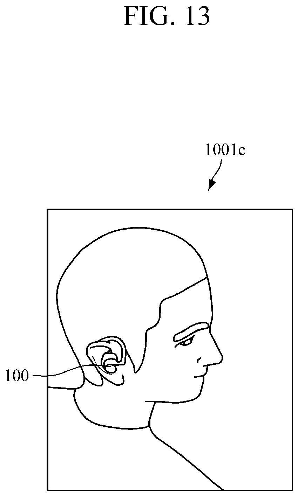

Referring to FIG. 13, the electronic device 1001 of FIG. 10 may be implemented as an ear wearable device 1001c.

The ear wearable device 100c may include a main body and an ear strap. A user may wear the electronic device 1001c of FIG. 13 by hanging the ear strap on a user's auricle. The ear strap may be omitted according to the type of ear wearable device 1001c. The main body may be inserted into the external auditory meatus. The apparatuses 100 and 7001 for estimating an analyte concentration may be mounted in the main body. The electronic device 1001c of FIG. 13 may provide a concentration estimation result as sounds to a user, or may transmit the concentration estimation result to an external device, e.g., a mobile device, a tablet PC, a personal computer, etc., through a communication module mounted in the main body.

While not restricted thereto, an example embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

The foregoing exemplary embodiments are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

**Claims**

1. An apparatus for estimating an analyte concentration, the apparatus comprising:

    a signal measurer comprising:

       an optical coherence tomography (OCT) device configured to emit an OCT signal to an object and receive the OCT signal reflected or scattered from the object; and
       a spectrometer configured to emit a spectrometer signal to the object and obtain a spectrum based on the spectrometer signal reflected or scattered from the object, and

    a processor configured to predict a path length distribution for each wavelength in a predetermined wavelength range, based on the OCT signal, and estimate a concentration of an analyte based on the predicted path length distribution and the obtained spectrum.

2. The apparatus of claim 1, wherein the OCT device comprises:

    a first light source configured to emit a first light;
    a beam splitter configured to split the first light into a first split light and a second split light;
    a reference mirror configured to reflect the first split light; and
    a first photodetector configured to detect coherent light generated from the first split that is reflected from the reference mirror, and the second split light that is emitted to and scattered from the object, and configured to convert the coherent light into the OCT signal.

3. The apparatus of claim 1, wherein the spectrometer comprises:

    a second light source configured to emit a second light; and
    a second photodetector configured to obtain the spectrum by detecting the second light that is emitted by the second light source and scattered from the object.

4. The apparatus of one of claims 1 to 3, wherein the processor is further configured to predict the path length distribution for each wavelength based on a scattering coefficient and a g-factor for each wavelength,
preferably, wherein the processor is further configured to obtain the scattering coefficient and the g-factor for each penetration depth of the spectrometer signal into the object, based on the OCT signal.

5. The apparatus of claim 4, wherein the processor is further configured to estimate the scattering coefficient for each penetration depth using a scattering coefficient function; and/or
wherein the processor is further configured to build a path length distribution database (DB) by using Monte Carlo simulation, preferably, wherein based on the g-factor and the estimated scattering coefficient for each wavelength, the processor is further configured to obtain the path length distribution for each wavelength from the built path length distribution DB.

6. The apparatus of one of claims 1 to 5, wherein the processor is further configured to obtain an absorption coefficient spectrum based on the path length distribution for each wavelength and the spectrum, and estimate the concentration of the analyte based on the absorption coefficient spectrum,
preferably, wherein the processor is further configured to determine an absorption coefficient for each wavelength, which allows an estimated spectrum, obtained based on the path length distribution for each wavelength and absorption coefficients at each wavelength, to converge on the spectrum obtained from the object.

7. The apparatus of claim 6, wherein the processor is further configured to remove a noise component, including temperature, from the obtained absorption coefficient spectrum,
preferably, wherein the processor is further configured to extract a noise component vector from the obtained absorption coefficient spectrum, based on at least one of Principal Component Analysis and Singular Value Decomposition.

8. The apparatus of claim 7, wherein the processor is further configured to remove noise from the absorption coefficient spectrum by using a noise removal method including a least square method.

9. The apparatus of one of claims 1 to 8, wherein the analyte comprises at least one of glucose, urea, lactate, triglyceride, total protein, cholesterol, and ethanol.

10. A method of estimating an analyte concentration, the method comprising:

   measuring an optical coherence tomography (OCT) signal from an object;
   obtaining a spectrum from the object;
   predicting a path length distribution for each wavelength in in a predetermined wavelength range, based on the OCT signal; and
   estimating a concentration of an analyte based on the predicted path length distribution and the obtained spectrum.

11. The method of claim 10, wherein the predicting of the path length distribution comprises predicting the path length distribution for each wavelength based on a scattering coefficient and a g-factor for each wavelength,
preferably, wherein the predicting of the path length distribution comprises obtaining the scattering coefficient and the g-factor for each penetration depth based on the OCT signal.

12. The method of claim 11, wherein the predicting of the path length distribution further comprises estimating the scattering coefficient for each wavelength based on the scattering coefficient for each penetration depth using a scattering coefficient function; and/or
wherein the predicting of the path length distribution further comprises building a path length distribution database (DB) by using Monte Carlo simulation, preferably, wherein the predicting of the path length distribution further comprises, based on the scattering coefficient and the g-factor for each wavelength, obtaining the path length distribution for each wavelength from the path length distribution DB.

13. The method of one of claims 10 to 12, wherein the estimating of the concentration of the analyte comprises obtaining an absorption coefficient spectrum based on the path length distribution for each wavelength and the spectrum, and estimating the concentration of the analyte based on the obtained absorption coefficient spectrum,
preferably, wherein the estimating of the concentration of the analyte comprises determining an absorption coefficient for each wavelength, which allows an estimated spectrum, obtained based on the path length distribution for each wavelength and absorption coefficients at each wavelength, to converge on the spectrum obtained from the object.

**14.** The method of claim 13, wherein the estimating of the concentration of the analyte comprises removing a noise component, including temperature, from the obtained absorption coefficient spectrum,
preferably, wherein the removing of the noise component comprises extracting a noise component vector based on at least one of Principal Component Analysis and Singular Value Decomposition.

**15.** The method of claim 14, wherein the removing of the noise component comprises removing noise from the absorption coefficient spectrum by using a noise removal method including a least square method.

# FIG. 1

# FIG. 2

# FIG. 3A

# FIG. 3B

FIG. 4A

# FIG. 4B

FIG. 4C

# FIG. 5A

INCIDENT
LIGHT
r = 0
z = 0

ABSORPTION
A (r, z)

SCATTERING
SPECTRUM
I (r)

dr

dz

r

z

SMP

# FIG. 5B

# FIG. 6

FIG. 7

700

710

SIGNAL MEASURER

720

PROCESSOR

750

COMMUNI-
CATION
INTERFACE

730

OUTPUT
INTERFACE

740

STORAGE

# FIG. 8

```
                        ┌─────────┐
                        │  START  │
                        └─────────┘
              ┌──────────────┴──────────────┐
              │ ╱810                         │ ╱820
      ┌───────────────────┐         ┌───────────────────┐
      │  OBTAIN OCT SIGNAL │         │  OBTAIN SPECTRUM  │
      │    FROM OBJECT     │         │    FROM OBJECT    │
      └───────────────────┘         └───────────────────┘
              │ ╱830                         │
      ┌───────────────────┐                 │
      │ PREDICT PATH LENGTH│                 │
      │  DISTRIBUTION FOR  │                 │
      │   EACH WAVELENGTH  │                 │
      └───────────────────┘                 │
              └──────────────┬──────────────┘
                             │ ╱840
             ┌───────────────────────────────┐
             │     ESTIMATE ANALYTE          │
             │  CONCENTRATION BY USING       │
             │ PATH LENGTH DISTRIBUTION      │
             │  FOR EACH WAVELENGTH          │
             │     AND A SPECTRUM            │
             └───────────────────────────────┘
                             │
                        ┌─────────┐
                        │   END   │
                        └─────────┘
```

# FIG. 9

START

↓

910

EXTRACT SCATTERING
COEFFICIENT AND G-FACTOR
FOR EACH DEPTH

920

OBTAIN SCATTERING
COEFFICIENT FOR EACH
WAVELENGTH BASED ON
SCATTERING COEFFICIENT
FOR EACH DEPTH

930

BUILD PATH LENGTH
DISTRIBUTION DB BY USING
MONTE CARLO SIMULATION

940

PREDICT PATH LENGTH
DISTRIBUTION FOR EACH
WAVELENGTH BY USING
SCATTERING COEFFICIENT
AND G-FACTOR FOR EACH
WAVELENGTH, AND PATH
LENGTH DISTRIBUTION DB

↓

END

# FIG. 10

ELECTRONIC DEVICE 1001

MEMORY 1030

MEMORY 1032
- VOLATILE MEMORY 1032
- NON-VOLATILE MEMORY 1034
  - INTERNAL MEMORY 1036
  - EXTERNAL MEMORY 1038

INPUT DEVICE 1050

SOUND OUTPUT DEVICE 1055

DISPLAY DEVICE 1060

BATTERY 1089

POWER MANAGEMENT MODULE 1088

PROCESSOR 1020
- MAIN PROCESSOR 1021
- AUXILIARY PROCESSOR 1023

COMMUNICATION MODULE 1090
- WIRELESS COMMUNICATION MODULE 1092
- WIRED COMMUNICATION MODULE 1094

SUBSCRIBER IDENTIFICATION MODULE 1096

AUDIO MODULE 1070

SENSOR MODULE 1076

HAPTIC MODULE 1079

CAMERA MODULE 1080

INTERFACE 1077

ANTENNA MODULE 1097

CONNECTION TERMINAL 1078

PROGRAM 1040
- APPLICATION 1046
- MIDDLEWARE 1044
- OPERATING SYSTEM 1042

NETWORK 1099

ELECTRONIC DEVICE 1004

ELECTRONIC DEVICE 1002

SERVER 1008

1000

1098

EP 3 981 329 A1

# FIG. 11

1001a

100

FIG. 12

FIG. 13

Europäisches
Patentamt

European
Patent Office

Office européen
des brevets

# EUROPEAN SEARCH REPORT

Application Number

EP 21 18 9621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/151976 A1 (TOMA CRISTIAN E [US]) 14 July 2005 (2005-07-14) * paragraphs [0030] – [0040], [0060] – [0063]; claims 1-28,44; figures 1-3 * ----- | 1-15 | INV. A61B5/145 A61B5/1455 A61B5/00 G16H10/40 |
| X | US 2019/015023 A1 (MONFRE STEPHEN LEONARD [US]) 17 January 2019 (2019-01-17) * claims 1-12; figures 1,2 * ----- | 1,10 | |
| A | WO 2015/082564 A1 (IMEC VZW [BE]) 11 June 2015 (2015-06-11) * claims 1-20; figures 1-4 * ----- | 1-15 | |
| A | US 2020/129095 A1 (LEE SO YOUNG [KR] ET AL) 30 April 2020 (2020-04-30) * paragraph [0070]; claims 1-13; figures 1-8 * ----- | 1-15 | |

TECHNICAL FIELDS
SEARCHED      (IPC)

A61B
G16H

The present search report has been drawn up for all claims

| Place of search | Date of completion of the search | Examiner |
|---|---|---|
| Munich | 12 January 2022 | Gentil, Tamara |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

## ANNEX TO THE EUROPEAN SEARCH REPORT
## ON EUROPEAN PATENT APPLICATION NO.

EP 21 18 9621

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2022

| Patent document cited in search report | | Publication date | Patent family member(s) | | Publication date |
|---|---|---|---|---|---|
| US 2005151976 | A1 | 14-07-2005 | US | 2005151976 A1 | 14-07-2005 |
| | | | WO | 2005058154 A1 | 30-06-2005 |
| US 2019015023 | A1 | 17-01-2019 | US | 2019015023 A1 | 17-01-2019 |
| | | | US | 2021378562 A1 | 09-12-2021 |
| | | | WO | 2019014629 A1 | 17-01-2019 |
| WO 2015082564 | A1 | 11-06-2015 | EP | 3076871 A1 | 12-10-2016 |
| | | | JP | 6507162 B2 | 24-04-2019 |
| | | | JP | 2017504362 A | 09-02-2017 |
| | | | US | 2017100064 A1 | 13-04-2017 |
| | | | WO | 2015082564 A1 | 11-06-2015 |
| US 2020129095 | A1 | 30-04-2020 | KR | 20200047981 A | 08-05-2020 |
| | | | US | 2020129095 A1 | 30-04-2020 |

EPO FORM P0459

For more details about this annex : see Official Journal of the European Patent Office, No. 12/82